# EUROPÄISCHE PATENTSCHRIFT

(11) **EP 2 250 994 B1**
(45) Veröffentlichungstag und Bekanntmachung des Hinweises auf die Patenterteilung: **12.10.2016**
(21) Anmeldenummer: 10162628.1
(22) Anmeldetag: 12.05.2010
(51) Int. Cl.: A61K 8/04, A61K 8/19, A61K 8/92, A61Q 19/06, A61Q 19/08, A61Q 19/10

(54) **Körperpeeling**
Body exfoliant
Gommage corporel

(30) Priorität: 14.05.2009 EP 09160272
(43) Veröffentlichungstag der Anmeldung: 17.11.2010
(73) Patentinhaber: Mischnick, Ruth, 53115 Bonn (DE)
(72) Erfinder: Mischnick, Ruth, 53115 Bonn (DE)
(74) Vertreter: dompatent von Kreisler Selting Werner - Partnerschaft von Patent- und Rechtsanwälten mbB

(56) Entgegenhaltungen:
- WO-A2-00/04867
- FR-A- 1 483 815
- "Pumpkin seed oil & Sea Salt Body Scrub", INTERNET CITATION, 13. Dezember 2007 (2007-12-13), Seite 1, XP002547883, Gefunden im Internet: URL:http://web.archive.org/web/20071223173 208/http://naturesbathandbeauty .com/pumpkin-seed-oil-sea-salt-scrub [gefunden am 2009-09-29]
- "Cranberry Pumpkin Salt Scrub", INTERNET CITATION, 4. November 2008 (2008-11-04), Seite 1, XP002547884, Gefunden im Internet: URL:http://www.naturalbeautyworkshop.com/m y_weblog/2008/11/cranberry-pump k.html [gefunden am 2009-09-29]
- DATABASE EPODOC EUROPEAN PATENT OFFICE, THE HAGUE, NL; Abstract zu GR1006297 "Body Peeling with natural products", XP002548064, & GR 1 006 297 B (SIGALA-CHARALABIDOU IFIGENEIA) 9. März 2009 (2009-03-09)

## Beschreibung

Die vorliegende Erfindung betrifft ein Peeling, ein Verfahren zur Herstellung eines Peelings und die Verwendung des Peelings.

Ein Peeling ist eine kosmetische oder dermatologische Behandlung, bei der oberflächliche Schichten der Haut flächig entfernt werden. Das übliche, kosmetisch eingesetzte Peeling ist eine oberflächliche Entfernung der obersten Hornschicht der Haut. Dieses oberflächliche "Peeling" wird auch als Mikrodermabrasion bezeichnet.

Grundsätzlich kann auch eine Abtragung der gesamten Hornschicht oder auch der Oberflächenschicht bis zur Kollagenschicht erfolgen. Diese als "mitteltiefes" oder "tiefes" Peeling bezeichneten Verfahren sind jedoch nur zu medizinischen Zwecken geeignet, da sie teilweise mehrere Wochen der Abheilung benötigen.

"Peeling" im Sinne dieser Anmeldung bedeutet eine oberflächliche Behandlung (Mikrodermabrasion), die der Anwender entweder selbst durchführt oder im Rahmen einer kosmetischen Behandlung durchführen lässt. Es umfasst selbstverständlich auch kosmetische Anwendungen, die beispielsweise durch Hautärzte angeboten werden.

Es existieren verschiedene Verfahren zum Peeling. Peeling ist möglich mit chemischen Substanzen, wie z.B. Fruchtsäuren und anderen Hydroxysäuren. Daneben ist es möglich, mit Hilfe von Lasern ein Peeling durchzuführen. Dies erfolgt im Wesentlichen zu medizinischen und nicht zu kosmetischen Zwecken.

Weiterhin weit verbreitet ist die Anwendung von mechanischen Peelingsubstanzen, beispielsweise durch Einsatz von Sand, Kunststoffpartikeln (insbesondere auf Polyethylenbasis) oder mit zerstoßenen oder zermahlenen Kernen von Pfirsichen und Aprikosen oder den Schalen von Walnüssen, etc.

Weitere abrasiv-wirkende Inhaltsstoffe sind Salze, wobei insbesondere verschiedene Meersalze eingesetzt werden.

WO 00/04867 beschreibt eine Hautreinigungsbehandlung enthaltend Salz, verschiedene Öle und ein Lecithin Phospholipid Emulgator.

Bei einem Peeling ist es wesentlich, dass das Abrasivum zwar die gewünschten Peelingeffekte erreicht, auf der anderen Seite jedoch nicht so aggressiv ist, dass die Haut durch die Behandlung Schaden aufnimmt.

Es besteht weiterhin ein Bedarf nach Körperpeelings, die zumindest einige Nachteile des Standes der Technik überwinden, insbesondere eine hohe Wirksamkeit bei gleichzeitiger Pflegeleistung zeigen.

Überraschenderweise kann die Aufgabe gelöst werden durch ein Peeling enthaltend:
- Salz
- Olivenöl
- Kürbiskernöl.

Erfindungsgemäß wird also ein Salz, beispielsweise ein Kochsalz oder Totes Meer Salz oder auch ein sonstiges beliebiges Meersalz, als Abrasivum eingesetzt. Die Körnung des Salzes bestimmt die abrasive Wirkung mit. Besonders geeignete mittlere Korngrößen liegen im Bereich von 1 bis 2 mm.

Das Gewichtsverhältnis Salz zu Öl liegt bevorzugt bei 10 Gew.-Teilen Salz zu 2 bis 8 Gewichtsteilen Öl, bevorzugt 3 bis 5 oder 6 bis 8 Gewichtsteilen.

Neben dem Olivenöl und Kürbiskernöl können grundsätzlich weitere Öle enthalten sein.

Ein geeignetes Gewichtsverhältnis von Kürbiskernöl zu den anderen Ölen (Olivenöl + gegebenenfalls weitere Öle) liegt im Bereich zwischen 1:3 und 1:5.

Bezogen auf 10 Gewichtsteile Salz sind bevorzugt mindestens 2 Gewichtsteile Olivenöl und 1 Gewichtsteil Kürbiskernöl enthalten.

Olivenöl besteht zu etwa 95 Gew.-% aus Triglyceriden, die zu etwa 78 Gew.-% einfach ungesättigte Fettsäuren (hauptsächlich Ölsäure) enthalten. Die weiteren Fettsäuren sind zu 12 Gew.-% mehrfach ungesättigte Fettsäuren und zu etwa 10% gesättigte Fettsäuren. Neben verschiedenen sekundären Pflanzenstoffen (Polyphenolen, Iridoiden) sind auch Vitamin E und Vitamin A enthalten.

Im Kürbiskernöl sind ca. 90 Gew.-% der Inhaltsstoffe Triglyceride. Der Großteil der Fettsäuren der Triglyceride sind ungesättigte Fettsäuren, etwa 70 bis 80 Gew.-% Linolsäure und 20 bis 30% verteilt auf Ölsäure, Palmitinsäure und Stearinsäure. Neben Mineralstoffen, Aminosäuren, Cucurbitin sind die Vitamine E, B1, B2, B6 sowie A, C und D enthalten.

Bevorzugt wird als Kürbiskernöl ein steierisches Kürbiskernöl eingesetzt. Dieses Öl wird aus dem steierischen Ölkürbis (Cucurbita pepo var. Styriaca) hergestellt. Dieser Kürbis besitzt keine verholzenden Samenschalen und erlaubt somit in einfacher Weise die Produktion von Öl. Er wird außer in der Steiermark auch in Ungarn, Slowenien, Russland angebaut.

Ein bevorzugtes weiteres Öl, das enthalten sein kann, ist Mandelöl. Mandelöl enthält neben den Fetten ca. 30% Eiweiß sowie 10% Glucose und Saccharose. Es enthält darüber hinaus Mineralstoffe wie Kalium, Calcium, Phosphor, Kupfer und Zink sowie Vitamine, insbesondere B-Vitamine.

Der Gehalt an Mandelöl liegt bevorzugt bei 0 bis 1 Gewichtsteilen pro 10 Gewichtsteile Salz.

Das erfindungsgemäße Körperpeeling kann weitere Hilfsstoffe enthalten wie Duftstoffe, Farbstoffe, Antioxidationsmittel. Bevorzugt enthält das Produkt keine Antioxidationsmittel. Weitere Inhaltsstoffe sind Lecithine (Phosphatidylcholine) und Derivate. Bevorzugt sind keine Emulgatoren, insbesondere lecithinbasierte Phosphorlipid Emulgatoren enthalten.

Das erfindungsgemäße Peeling wird hergestellt durch das Vermischen von Salz mit Olivenöl, Kürbiskernöl sowie gegebenenfalls weiteren Ölen und Hilfsstoffen.

Das erfindungsgemäße Peeling eignet sich insbesondere als Körperpeeling, das vor dem Baden oder Duschen eingesetzt wird. Durch das Peeling werden überraschende kosmetische Effekte erzielt, insbesondere Hautbildverfeinerung, Faltenrückbildung und/oder Celluliteverringerung. Insbesondere bei regelmäßiger Anwendung kann eine überraschend starke Wirkung auf Cellulite erreicht werden. Zusätzlich zeigt sich eine Verminderung von Altersflecken bei regelmäßigem Einsatz.

Die Erfindung wird durch die folgenden Beispiele näher erläutert:

### Beispiel 1

500 g Kochsalz, 200 ml Olivenöl und 50 ml Kürbiskernöl wurden miteinander vermengt und dreimal wöchentlich vor dem Duschen als Körperpeeling eingesetzt. Es zeigte sich nach einwöchiger Anwendung eine signifikante Verbesserung der Haut, insbesondere hinsichtlich der Cellulite.

### Beispiel 2

500 g Totes Meer Salz wurden mit 200 ml Olivenöl, 45 ml Kürbiskernöl und 5 ml Mandelöl vermengt und ebenfalls als Körperpeeling eingesetzt.

Durch den Zusatz von Mandelöl verbessert sich die pflegende Wirkung des Peelings noch etwas.

Die regelmäßige Anwendung führte zu einer deutlichen Faltenrückbildung.

## Patentansprüche

1. Peeling enthaltend
- Salz
- Olivenöl
- Kürbiskernöl,
wobei auf 10 Gew.-Teile Salz 2 bis 8 Gew.-Teile Öl enthalten sind.

2. Peeling nach Anspruch 1, **dadurch gekennzeichnet, dass** zusätzlich weitere Öle, insbesondere Mandelöl enthalten ist.

3. Peeling nach Anspruch 1 oder 2, **dadurch gekennzeichnet, dass** das Kürbiskernöl aus steirischem Kürbis gewonnen wurde.

4. Peeling nach einem der Ansprüche 1 bis 3, **dadurch gekennzeichnet, dass** auf ein Gewichtsteil Kürbiskernöl 3 bis 5 Gewichtsteile Mandelöl und gegebenenfalls weitere Öle enthalten sind.

5. Peeling nach einem der Ansprüche 1 bis 4, **dadurch gekennzeichnet, dass** das Salz ausgewählt wird aus Kochsalz und Totes Meer Salz.

6. Peeling nach einem der Ansprüche 1 bis 5, **dadurch gekennzeichnet, dass** die mittlere Korngröße des Salzes zwischen 1 bis 2 mm liegt.

7. Peeling nach einem der Ansprüche 1 bis 6, **dadurch gekennzeichnet, dass** zusätzlich Hilfsstoffe wie Duftstoffe, Farbstoffe, Antioxidationsmittel enthalten sind.

8. Verfahren zur Herstellung eines Peelings nach einem der Ansprüche 1 bis 7, **dadurch gekennzeichnet, dass** Salz mit Olivenöl, Kurbiskernöl sowie gegebenenfalls weiteren Ölen und Hilfsstoffen vermengt wird.

9. Verwendung eines Peelings nach einem der Ansprüche 1 bis 7, als Körperpeeling vor dem Duschen oder Baden.

10. Verwendung eines Peelings nach mindestens einem der Ansprüche 1 bis 7 zur Hautbildverfeinerung, Faltenrückbildung und/oder Celluliteverringerung.

11. Verwendung eines Peelings nach mindestens einem der Ansprüche 1 bis 7 zur Faltenrückbildung.

12. Verwendung eines Peelings nach mindestens einem der Ansprüche 1 bis 7 zur Celluliteverringerung.

## Claims

1. A peeling composition, comprising
- salt
- olive oil
- pumpkin seed oil,
wherein from 2 to 8 weight parts of oil are contained per 10 weight parts of salt.

2. The peeling composition according to claim 1, **characterized in that** further oils, especially almond oil, are additionally contained.

3. The peeling composition according to claim 1 or 2, **characterized in that** said pumpkin seed oil has been obtained from Styrian oil pumpkin.

4. The peeling composition according to any of claims 1 to 3, **characterized in that** from 3 to 5 weight parts of almond oil and optionally further oils are contained per one weight part of pumpkin seed oil.

5. The peeling composition according to any of claims 1 to 4, **characterized in that** said salt is selected from table salt and Dead Sea salt.

6. The peeling composition according to any of claims 1 to 5, **characterized in that** the mean grain size of the salt is from 1 to 2 mm.

7. The peeling composition according to any of claims 1 to 6, **characterized in that** auxiliary agents, such as perfumes, colorants, antioxidants, are additionally contained.

8. A process for producing a peeling composition according to any of claims 1 to 7, **characterized in that** salt is mixed with olive oil, pumpkin seed oil, and optionally further oils and auxiliary agents.

9. Use of a peeling composition according to any of claims 1 to 7 as a body scrub before showering or taking a bath.

10. Use of a peeling composition according to at least one of claims 1 to 7 for skin texture refining, wrinkle regression and/or cellulite reduction.

11. Use of a peeling composition according to at least one of claims 1 to 7 for wrinkle regression.

12. Use of a peeling composition according to at least one of claims 1 to 7 for cellulite reduction.

## Revendications

1. Peeling, contenant
- du sel
- de l'huile d'olive
- de l'huile de pépins de courge,
2 à 8 parties en poids d'huile étant contenues pour 10 parties en poids de sel.

2. Peeling selon la revendication 1, **caractérisé en ce qu'**il contient en plus d'autres huiles, en particulier de l'huile d'amande.

3. Peeling selon la revendication 1 ou 2, **caractérisé en ce que** l'huile de pépins de courge a été obtenue à partir de courge de Styrie.

4. Peeling selon l'une des revendications 1 à 3, **caractérisé en ce que** 3 à 5 parties en poids d'huile d'amande et éventuellement d'autres huiles sont contenues pour une partie en poids d'huile de pépins de courge.

5. Peeling selon l'une des revendications 1 à 4, **caractérisé en ce que** le sel est sélectionné parmi du sel de cuisine et du sel de la mer Morte.

6. Peeling selon l'une des revendications 1 à 5, **caractérisé en ce que** la granulométrie moyenne du sel est comprise entre 1 et 2 mm.

7. Peeling selon l'une des revendications 1 à 6, **caractérisé en ce qu'**il contient en plus des adjuvants tels que des parfums, des colorants, des antioxydants.

8. Procédé de production d'un peeling selon l'une des revendications 1 à 7, **caractérisé en ce que** du sel est mélangé à de l'huile d'olive, de l'huile de pépins de courge ainsi éventuellement qu'à d'autres huiles et adjuvants.

9. Utilisation d'un peeling selon l'une des revendications 1 à 7, en tant que peeling corporel avant la douche ou le bain.

10. Utilisation d'un peeling selon au moins l'une des revendications 1 à 7 pour l'affinement du grain de la peau, la réduction des rides et/ou la diminution de la cellulite.

11. Utilisation d'un peeling selon au moins l'une des revendications 1 à 7 pour la réduction des rides.

12. Utilisation d'un peeling selon au moins l'une des revendications 1 à 7 pour la diminution de la cellulite.
